# EUROPEAN PATENT SPECIFICATION

(11) **EP 0 192 480 B1**
(45) Date of publication and mention of the grant of the patent: **23.11.1994**
(21) Application number: 86301210.0
(22) Date of filing: 20.02.1986
(51) Int. Cl.: C07C 215/28, C07C 321/24, C07C 317/00

(54) **Bis(3-aminophenoxy) aromatics and method of preparing the same**
Bis(3-aminophenoxy)aromaten und Verfahren zu ihrer Herstellung
Bis(amino-3 phénoxy)aromatiques et procédé pour leur préparation

(30) Priority: 22.02.1985 JP 32568/85; 28.05.1985 JP 113237/85; 28.06.1985 JP 140408/85; 22.08.1985 JP 183039/85; 17.09.1985 JP 203557/85; 25.09.1985 JP 210266/85; 24.12.1985 JP 289334/85
(43) Date of publication of application: 27.08.1986
(73) Proprietor: MITSUI TOATSU CHEMICALS, Inc., Chiyoda-Ku Tokyo 100 (JP)
(72) Inventor: Yoshikawa, Yukihiro, Zushi-shi Kanagawa-ken (JP); Yamaguchi, Keizaburo, Kawasaki-shi Kanagawa-ken (JP); Sugimoto, Kenichi, Yokohama-shi Kanagawa-ken (JP); Tanabe, Yoshimitsu, Yokohama-shi Kanagawa-ken (JP); Yamaguchi, Akihiro, Kamakura-shi Kanagawa-ken (JP)
(74) Representative: Rackham, Anthony Charles

(56) References cited:
- US-A- 4 196 144
- US-A- 4 203 922
- US-A- 4 222 962
- PATENT ABSTRACTS OF JAPAN, unexamined applications, C section, vol. 3, no. 63, May 30, 1979 THE PATENT OFFICE JAPANESE GOVERNMENT page 117 C 47.
- LIEBIGS ANNALEN DER CHEMIE, vol. 740, 1970, Weinheim J. SCHRAMM et al. "Nucleophile aromatische Substitution mit Aminophenolaten " pages 167-179.

## Description

The present invention relates to bis(3-aminophenoxy) derivatives of aromatic or bridged aromatic hydrocarbons (hereinafter abbreviated as bis(3-aminophenoxy)-derivatives) and the method for preparing the same which comprises conducting a condensation reaction of m-dinitrobenzene with dihydroxy-derivatives of aromatic or bridged aromatic hydrocarbons (hereinafter abbreviated as dihydroxy-derivatives) in dipolar aprotic solvents in the presence of bases, and successively reducing the bis(3-nitrophenoxy) derivatives of aromatic or bridged aromatic hydrocarbons (hereinafter abbreviated as bis(3-nitrophenoxy)-derivatives) obtained thereby.

Regarding bis(3-aminophenoxy)-derivatives represented by the following general formula: 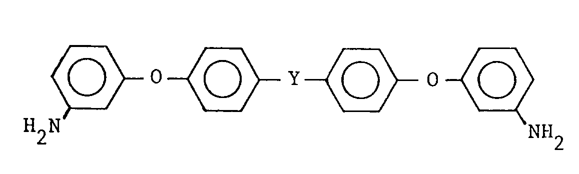
where Y is a C₁-C₁₀ hydrocarbon, -C(CF₃)₂-, -CO-, -S-, -SO-, -SO₂-, or -O-,
and having amino groups at the meta positions of the ether linkage, the compound 4,4-bis(3-aminophenoxy)diphenylsulfone, where Y is -SO₂- in the general formula above, is known to have been prepared by the condensation of m-aminophenol with 4,4'-dichlorodiphenylsulfone in dimethylsulfoxide in the presence of potassium hydroxide (J. Schramm et al., Liebigs Ann. Chem., 740, 169 (1970), TOKKOSHO 58-35990). Preparation methods have, however, not previously been known for the compounds having the divalent group where Y is -C(CF₃)₂-, -C(CH₃)₂-, -CO-, -S-, -SO-, or -O-.

An object of the present invention is to provide a novel method for cheaply and industrially preparing bis(3-aminophenoxy)-derivatives and to provide useful and novel bis(3-aminophenoxy)-derivatives as starting materials for the production of high-temperature stable polymers.

An intensive investigation was conducted by the inventors of this invention to achieve the above object. Unexpectedly, a new fact was found that bis(3-nitrophenoxy)-derivatives can easily be derived from m-dinitrobenzene by condensation with dihydroxy-derivatives. The bis(3-aminophenoxy)-derivatives desired can then been achieved by reducing the bis(3-nitrophenoxy)-derivative Novel bis (3-aminophenoxy)-derivatives which have never before been prepared can be provided by the new method.

Accordingly, the present invention provides a method for preparing a bis(3-aminophenoxy)-derivative of an aromatic or bridged aromatic hydrocarbon represented by the following general formula: 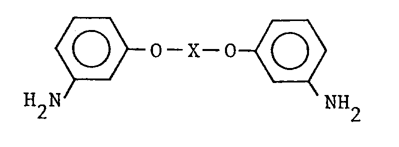
where X is 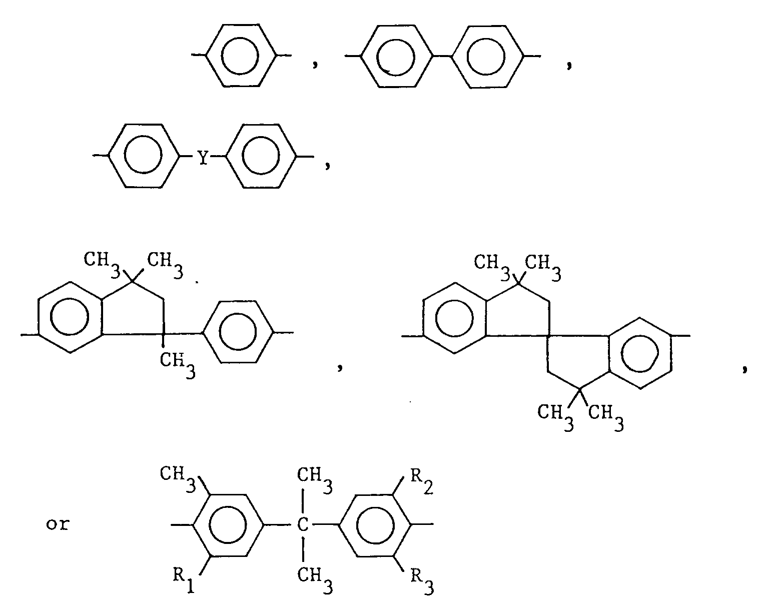
where Y is a C₁-C₁₀ hydrocarbon, -C(CF₃)₂-, -CO-, -S-, -SO-, -SO₂-, or -O-,
and R₁, R₂, R₃ are H, or CH₃,
which comprises conducting a condensation reaction of m-dinitrobenzene in a dipolar aprotic solvent in the presence of a base with a dihydroxy-derivative of an aromatic or bridged aromatic hydrocarbon represented by the following general formula:

HO-X-OH

where X is the same as above,
and reducing the resultant bis(3-nitrophenoxy)-derivative of the aromatic or bridged aromatic hydrocarbon represented by the following general formula: 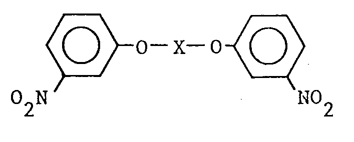
where X is the same as above.

Many examples are known of the substitution reaction of aromatic nitro compounds, activated by o- or p-located electron-withdrawing groups, with alcohols and phenols. (H. M. Relles et al., J. Polym. Sci., Polym. Chem. Ed., 15, 2441 (1977); J. R. Beck et al., J. Org. Chem., 39, 1839 (1974); T. Takekoshi et al., J. Polym. Sci., Polym. Chem. Ed., 18, 3069 (1980); J. R.Beck, Tetrahedron, 34, 2057 (1978)).

Only a few examples, however, are known of the substitution reaction of inactive nitro compounds, such as in m-dinitrobenzene, with alcohols, and only one example is found of the reaction with phenols.

Thus:
(1) m-Nitroanisole was prepared by reacting m-dinitrobenzene with sodium methoxide at 25°C for 16 hours in hexamethylphosphotriamide solvent (N. Kornblum et al., J. Org. Chem., 41, 1560 (1976)).
(2) m-Nitrophenyl alkyl ether was prepared by reacting m-dinitrobenzene with alkali metal alkoxide in dipolar aprotic solvents in the presence of macro-cyclic polyethers (Toyota et al., TOKKAISHO 54-39030).
(3) m-Nitroanisole was prepared by reacting m-dinitrobenzene with sodium methoxide in chlorobenzene in the presence of phase-transfer catalysts (F. Montanari et al., Chem. & Ind., (1982), 412).
(4) The possibility of preparing 3-nitrodiphenylethers by reacting m-dinitrobenzene with alkali metal phenolates in polar solvents in the presence of macro-cyclic polyethers is described without detail (TOKKAISHO 54-39030)
In these methods, however, special solvents such as hexamethylphosphotriamide or special reagents such as expensive and toxic crown ethers are required for accelerating the reaction. Hence the substitution reaction of m-dinitrobenzene with phenols was assumed to present considerably difficulty, and no examples have so far been found of the reaction under normal conditions of industry.

According to the method of this invention, a variety of bis(3-aminophenoxy)-derivatives are prepared by performing the condensation reaction of m-dinitrobenzene with dihydroxy-derivatives, which has so far been supposed to be difficult.

Therefore, the method of the present invention is very suitable for industrial application.

The dihydroxy-derivatives used in the present invention are represented by the following general formula:

HO-X-OH

where X is 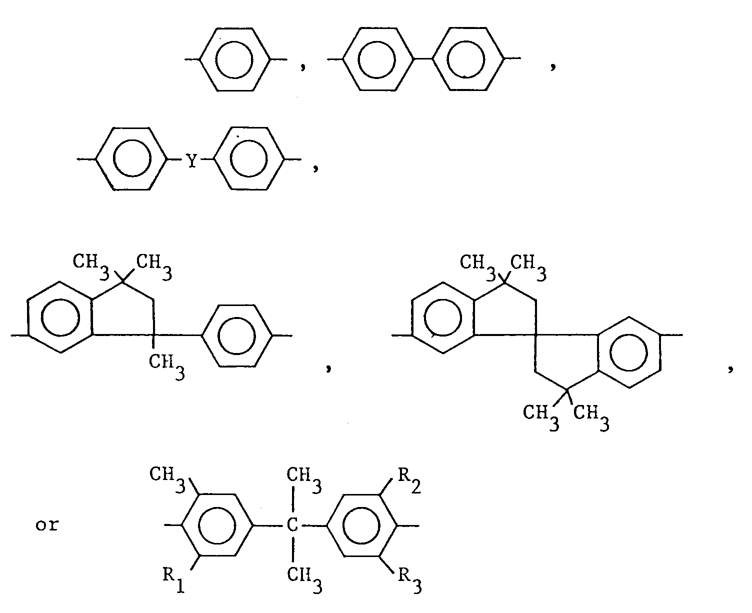
where Y is a C₁-C₁₀ hydrocarbon, -C(CF₃)₂-, -CO-, -S-, -SO-, -SO₂-, or -O-,
and R₁, R₂, R₃ are H, or CH₃.

Specifically, the dihydroxy-derivatives represented by the follwing general formula: 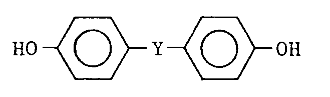
where Y is a C₁-C₁₀ hydrocarbon, -C(CF₃)₂-, -CO-, -S-, -SO-, -SO₂-, or -O-,
include, for example, 4,4'-dihydroxydiphenylmethane, 2,2-bis(4-hydroxyphenyl)propane, 2,4-bis(4-hydroxyphenyl)-2-methylpentane, 2,4-bis(4-hydroxyphenyl)-4-methyl-1-pentene, 2,2-bis(4-hydroxyphenyl)1,1,1,3,3,3-hexafluoropropane, 4,4'-dihydroxybenzophenone, 4,4'-dihydroxydiphenylsulfide, 4,4'-dihydroxydiphenylsulfoxide, 4,4'-dihydroxydiphenylsulfone and 4,4'-dihydroxydiphenylether.

The examples of dihydroxy-derivatives also include hydroquinone, 4,4'-dihydroxybiphenyl, 1-(4-hydroxyphenyl)-1,3,3-trimethyl-6-hydroxyindan, 6,6'-dihydroxy-3,3,3',3'-tetramethyl-1,1'-spirobiindan and further include 2,2-bis(4-hydroxyphenyl)propane derivatives represented by the following general formula: 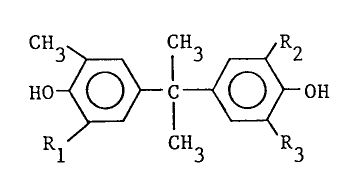
where R₁, R₂ and R₃ are H, or CH₃,
such as 2-(4-hydroxy-3-methylphenyl)-2-(4'-hydroxyphenyl)propane, 2-(4-hydroxy-3,5-dimethylphenyl)-2-(4'-hydroxyphenyl)propane, 2,2-bis(4-hydroxy-3-methylphenyl)propane and 2,2-bis(4-hydroxy-3,5-dimethylphenyl)propane.

Bis(3-aminophenoxy)-derivatives obtained by the method of this invention and represented by the following general formula: 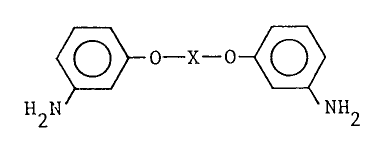
where X is the same as above,
include, for example, 4,4'-bis(3-aminophenoxy)diphenylmethane, 2,2-bis[4-(3-aminophenoxy)phenyl]propane, 2,4-bis[4-(3-aminophenoxy)phenyl]-2-methylpentane, 2,4-bis[4-(3-aminophenoxy)phenyl]-4-methyl-1 pentene, 2,2-bis[4-(3-aminophenoxy)phenyl]-1,1,1,3,3,3-hexafluoropropane, 4,4'-bis(3-aminophenoxy)benzophenone, 4,4'-bis(3-aminophenoxy)diphenylsulfide, 4,4'-bis(3-aminophenoxy)diphenylsulfoxide, 4,4'-bis(3-aminophenoxy)diphenylsulfone and 4,4'-bis(3-aminophenoxy)diphenylether.

Further bis(3-aminophenoxy)-derivatives include, for example, 1,4-bis(3-aminophenoxy)benzene, 4,4'-bis(3-aminophenoxy)biphenyl, 1-[4-(3-aminophenoxy)phenyl]-1,3,3-trimethyl-6-(3-aminophenoxy)indan and 6,6'-bis(3-aminophenoxy)-3,3,3',3'-tetramethyl-1,1'-spirobiindan.

Still further bis(3-aminophenoxy)-derivatives are methyl substituted derivatives of 2,2-bis[4-(3-aminophenoxy)phenyl]propane represented by the following general formula: 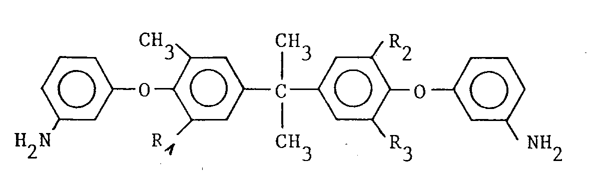
where R₁, R₂ and R₃ are H, or CH₃,
and include, for example, 2-[3-methyl-4-(3-aminophenoxy)phenyl]-2-[4-(3-aminophenoxy)phenyl]propane, 2-[3,5-dimethyl-4-(3-aminophenoxy)-phenyl]-2-[4-(3-aminophenoxy)phenyl]propane, 2,2-bis[3-methyl-4-(3-aminophenoxy)phenyl]propane and 2,2-bis[3,5-dimethyl-4-(3-aminophenoxy)phenyl]propane.

The above stated derivatives are new compounds except for 1,4-bis(3-aminophenoxy)benzene.

In the method of this invention, the quantities used of the starting materials, such as the dihydroxy-derivatives and m-dinitrobenzene, are not limited, but normally m-dinitrobenzene is employed in the range of 1.5-4.0 times by mol of the dihydroxy-derivative.

The bases used in the method of this invention may be alkali metal oxides, hydroxides, carbonates, hydrogen carbonates, hydrides or alkoxides, and include, for example, sodium oxide, lithium oxide potassium hydroxie, sodium hydroxide, lithium hydroxide, potassium carbonate, sodium carbonate, potassium hydrogen carbonate, sodium hydrogen carbonate, sodium hydride, sodium methoxide, sodium ethoxide, potassium tert-butoxide and potassium propoxide. Among these bases, alkali metal carbonates and hydrogen carbonates are preferably used. These bases may be used singly or in combinations of two or more.

The quantity used of these bases is normally in the range of 1-5 times by mol (1-10 equivalents), and preferably 1.5-3 times by mol of the dihydroxy-derivative.

Reaction accelerators which may be used when needed in the method of this invention, include quaternary ammonium salts, quaternary phosphonium salts, nitrogen containing aliphatic polyethers, macro-cyclic polyethers such as crown ether, nitrogen containing macro-cyclic polyethers such as cryptate, phase-transfer catalysts such as polyethyleneglycol and its alkyl ethers, copper powder and copper salts. Among the reaction accelerators, nitrogen-containing aliphatic polyethers represented by the following general formula:

N[-CH₂-CH₂-O(-CH₂-CH₂-O)ₙ-R]₃

where R is a C₁-C₄ alkyl radical and n is the integer 1 or 2, can, in particular, still further accelerate the reaction. of the present invention.

The nitrogen containing aliphatic polyethers can be easily available (TOKKOSHO 57-37580, 58-34464). The polyethers of above general formula include, for example, tris(3,6-dioxaheptyl)amine, tris(3,6,9-trioxadecyl)amine, tris(3,6-dioxaoctyl)amine, tris(3,6,9-trioxaundecyl)amine, tris(3,6-dioxanonyl)amine, tris(3,6,9-trioxadodecyl)amine, tris(3,6-dioxadecyl)amine, and tris(3,6,9-trioxatridecyl)amine. Tris(3,6-dioxaheptyl)amine is most frequently used for industrial application.

The quantity in use of these nitrogen containing aliphatic polyethers is unlimited. The catalytic amounts are normally sufficient and in the range of 0.1-10%, preferably 0.2-5% by mol of dihydroxy-derivatives The effect on accelerating the reaction depends upon the quantity and performance of applied catalysts. When the catalysts are 1% by mol of dihydroxy-derivatives, reaction time can be reduced to a half to one tenth.

The solvents used in the process of this invention are the dipolar aprotic solvents and include, for example, N,N-dimethylformamide, N,N-dimethylacetamide, dimethylsulfoxide, dimethylsulfone, sulfolane, 1-methyl-2-pyrrolidinon, 1,3-dimethyl-2-imidazolidinon, N,N,N',N'-tetramethylurea, hexamethylphosphotriamide, and 1,3-dimethyl-3,4,5,6-tetrahydro-2(H)pyrimidine The quantity of solvents is not limited in particular and normally 1-15 times, preferably 3-10 times by weight of the raw materials.

On performing the reaction, procedures such as charging of the raw materials are not restricted. There are some procedures such as, for example:
(1) Specified quantities of bisphenol derivative, base and solvent are previously charged to form an alkali phenolate of the dihydroxy-derivative before adding the m-dinitrobenzene to carry out the reaction.
(2) All the starting materials including the m-dinitrobenzene are charged at the start and the reaction thus performed. Those procedures are not subject to restriction because either of them will perform the reaction.

When an alkali metal oxide or hydroxide is used as the base, water generated in the reaction system can be removed, for example, gradually by introducing a nitrogen stream, or by azeotropic distillation with a small quantity of benzene, toluene, xylene, chlorobenzene or the like. When carbonate or hydrogen carbonate is used, no particular dehydration procedure is required.

Reaction temperatures are normally in the range of 100-240°C and preferably 120-180°C. Reaction time is normally 5-30 hours.

At the end of the reaction, the reaction mixture is heated to distill off the solvents, or used as it is, and poured into water. The crude product thus obtained is used for the following reduction step as it is, or after purification when needed.

The reduction process employed for the reducing reaction is not restricted in particular, conventional methods for reducing nitro groups to amino groups being applicable (for example, as in A New Experimental Chemistry Course, vol 15, Oxidation and Reduction (II), from Maruzen (1977)). Catalytic reduction or hydrazine reduction is preferred for industrial application.

As to the catalyst used for catalytic reduction, conventional metal reducing catalysts can be employed which include, for example, nickel, palladium, platinum, rhodium, ruthenium, cobalt and copper. Palladium catalyst is the most preferred for industrial application. The catalyst can be used in a metallic state and is normally employed supported on a carrier medium such as carbon, barium sulfate, silica gel, alumina and zeolite. Nickel, cobalt and copper can also be used in the form of Raney catalysts.

The quantity of catalyst used is not limited in particular and may be in the range of 0.01-10% by weight as metal on the dihydroxy-derivative. Normally, the range is 2-8% by weight when the catalyst used is the metal alone, and 0.1-5% by weight when the catalyst is on a carrier.

The solvents used for the reducing reaction are not limited in particular, unless they are active in the reaction, and include, for example, alcohols such as methanol, ethanol and isopropylalcohol; glycols such as ethyleneglycol and propyleneglycol; ethers such as diethylether, dioxane, tetrahydrofuran and ethyleneglycol monomethylether; aliphatic hydrocarbons such as hexane and cyclohexane; aromatic hydrocarbons such as benzene, toluene and xylene; esters such as ethyl acetate and butyl acetate; halogenated hydrocarbons such as dichloromethane, chloroform, tetrachloromethane, 1,2-dichloroethane, 1,1,2-trichloroethane and tetrachloroethane; N,N-dimethyl formamide and dimethylsulfoxide. When water immiscible solvents are used and the reaction speed is slow, the reaction can be accelerated by adding conventional phase-transfer catalysts such as quaternary ammonium salts or quaternary phosphonium salts. The quantity of solvent is not critical and should be sufficient to suspend or dissolve the reaction material. A sufficient quantity of solvent is normally 0.5-10 times by weight of the reaction material.

The reducing reaction temperatures are not limited in particular. The preferable range is generally 20-200°C and particularly 20-100°C. The reaction pressure is normally about ambient to 50 kg/cm².

The reaction is normally conducted by dissolving or suspending the reaction material in the solvent before adding the catalyst, followed by introducing hydrogen to perform reduction at the specified temperature under stirring. The end point of the reaction can be determined by the volume of hydrogen absorbed as well as by thin-layer chromatography or high speed liquid chromatography.

On the other hand, in reduction with hydrazine, it is sufficient to use a small excess of hydrazine above the theoretical quantity. Preferably, 1.2-2 times are used to perform the reduction.

The same metal catalysts as those generally in use for catalytic reduction can again be used. Industrially preferred are palladium/carbon, platinum/carbon and ferric chloride absorbed on active carbon. The quantity of such catalyst used may be in the range of 0.05-30% by weight as metal on the bis(3-nitrophenoxy)-derivative.

The solvents used in reduction with hydrazine are the same as for catalytic reduction. The reaction temperatures are not limited in particular and the preferred range is 20-150°C and move particularly 40-100°C.

The reaction is normally conducted by dissolving or suspending the reaction material in the solvent before adding the catalyst, followed by dropping hydrazine to perform the reduction at the specified temperature under stirring. The end point of the reaction can be determined by thin-layer chromatography or high speed liquid chromatography.

At the end of the reaction, the resulting mixture is hot-filtered to remove the catalyst, and the solvents are distilled off when necessary. The desired bis(3-aminophenoxy)-derivatives are obtained as crude products which can be purified by recrystallization or by isolating in the form of hydrochloride.

### EXAMPLES

The techniques of the invention will be illustrated further by means of the following Examples.

### Example 1

A 3 l glass reaction vessel was charged with 186 grams (1.0 mol) of 4,4'-dihydroxybiphenyl, 438 grams (2.6 mols) of m-dinitrobenzene, 363 grams of potassium carbonate and 2,000 ml of N,N-dimethylformamide. The mixture was reacted at 145-150°C for 16 hours.

After the end of the reaction, the resultant mixture was cooled and filtered to remove KNO₂. The filtrate was heated in a vacuum to distill off the solvent, and cooled to 65°C, this being followed by adding 2,000 ml of methanol, and stirring for an hour. The separated crystals were filtered, washed with water and methanol successively, and dried to obtain 426 grams (99.5% yield) of 4,4'-bis(3-nitrophenoxy)biphenyl as brown crystals. The purity was 91.0% based on liquid chromatography.

A portion of the crude crystals was recrystallized from dimethylsulfoxide to give the pure compound as light yellow crystals having a melting point of 134-136°C. Analytical results were as follows.

| Elemental analysis (C₂₄H₁₆N₂O₆) | | | |
|---|---|---|---|
| | C | H | N |
| Calculated (%) | 67.3 | 3.74 | 6.54 |
| Found (%) | 67.34 | 3.70 | 6.48 |

- MS:: 428 (M⁺)
- IR (KBr.cm⁻¹):: 1520, 1350 (nitro group),
1240 (ether linkage)

In the next step, a 1 l glass reaction vessel was charged with 100 grams (0.23 mol) of crude 4,4'-bis(3-nitrophenoxy)biphenyl, 10 grams of active carbon, 1 gram of ferric chloride hexahydrate and 500 ml of ethyleneglycol monomethylether. The mixture was refluxed with stirring for 30 minutes, followed by dropwise addition of 46 grams (0.92 mol) of hydrazine hydrate at 70-80°C during 3 hours. At the end of the addition, the resulting mixture was stirred at 70-80°C for 5 hours to complete the reaction. The reaction product was cooled, filtered to remove the catalyst, and poured into 500 ml of water. The separated crystals were filtered, dissolved into a hot mixture of 48 grams of 35% hydrochloric acid and 540 ml of 50% aqueous iso-propylalcohol (IPA) solution, and allowed to cool. The separated 4,4'-bis(3-aminophenoxy)biphenyl hydrochloride was filtered, dissolved by warming in 540 ml of 50% aqueous IPA solution, and filtered again after adding 5 grams of active carbon.

The filtrate was neutralized with aqueous ammonia and the separated crystals were filtered, washed with water and dried to obtain 72.0 grams (85% yield) of 4,4'-bis(3-aminophenoxy)biphenyl as colorless crystals having a melting point of 144-146°C. The analytical results were as follows.
Purity: 99.6% (based on high speed liquid chromatography)

| Elemental analysis (C₂₄H₂₀N₂O₂) | | | |
|---|---|---|---|
| | C | H | N |
| Calculated (%) | 78.26 | 5.43 | 7.61 |
| Found (%) | 78.56 | 5.21 | 7.66 |

MS: 368 (M⁺), 340, 184
IR (KBr.cm⁻¹): 3400 and 3310 (amino group),
1240 (ether linkage)

### Example 2

A 1 l sealed glass vessel was charged with 100 g (0.23 mol) of the crude 4,4'-bis(3-nitrophenoxy)biphenyl obtained in Example 1, 1 g of 5% Pd/C (made by Japan Engelhardt Co.) and 350 ml of ethyleneglycol monomethylether. Hydrogen was introduced at 60-65°C with vigorous stirring. Absorption of hydrogen was stopped after 8 hours at the end of any reaction.

The same after-treatment and purification as in Example 1 were conducted to obtain 70.3 grams (83% yield) of 4,4'-bis(3-aminophenoxy)biphenyl as colorless crystals having a melting point of 144-146°C and a purity of 99.3% based on high speed liquid chromatography.

### Example 3

The reaction procedure of Example 1 was repeated except 3.2 grams (0.01 mol) of tris(3,6-dioxaheptyl)amine was added and reacted for 2 hours at 145-150°C.

The same after-treatment as Example 1 was conducted to obtain 426 grams (99.5% yield) of 4,4'-bis(3-nitrophenoxy)biphenyl as yellow brown crystals having a purity of 97.5% based on liquid chromatography.

### Example 4

A 5 l glass reaction vessel was charged with 343 grams (1.5 mols) of 2,2-bis(4-hydroxyphenyl)propane, 605 grams (3.6 mols) of m-dinitrobenzene, 498 grams (3.6 mols) of potassium carbonate and 3.4 l of N,N-dimethylformamide. The mixture was reacted at 145-150°C for 10 hours, cooled after the end of the reaction, and filtered to remove KNO₂. The filtrate was distilled in a vacuum to remove the solvent, cooled to 65°C, mixed with 1.8 l of methanol and stirred for an hour. The separated crystals were filtered, washed with water and methanol successively, and dried to obtain 660 grams (93.5% yield) of 2,2'-bis[4-(3-nitrophenoxy)phenyl]propane as yellow brown crystals, having a purity of 93% based on liquid chromatography.

A portion of the crude crystals was recrystallized from ethyleneglycol monomethylether. Pure crystals were obtained as light yellow prisms having a melting point of 111-113°C and their analysis was as follows.

| Elemental analysis (C₂₇H₂₂N₂O₆) | | | |
|---|---|---|---|
| | C | H | N |
| Calculated (%) | 68.93 | 4.71 | 5.96 |
| Found (%) | 69.05 | 4.65 | 5.94 |

- MS:: 470 (M⁺), 455 (M-CH₃)⁺
- IR (KBr.cm⁻¹):: 1520 and 1340 (nitro group)

In the second step, a 500 ml glass reaction vessel was charged with 100 grams (0.21 mol) of 2,2-bis[4-(3-nitrophenoxy)phenyl]propane, 10 grams of active carbon, 1 gram of ferric chloride hexa-hydrate and 300 ml of ethyleneglycol monomethylether. The mixture was refluxed with stirring for 30 minutes, followed by addition by dropping of 42 grams (0.84 mol) of hydrazine hydrate at 70-80°C during 2 hours, and the mixture was stirred for a further 5 hours at 70-80°C. The resultant reaction mixture was cooled, and filtered to remove the catalyst and 150 ml of ethyleneglycol monomethylether was distilled off. To the residual matter, 270 grams of 20% aqueous hydrogen chloride solution and 30 grams of sodium chloride were added, and the mixture cooled to 20-25°C with stirring. The separated crystals were filtered, dissolved into 30% aqueous IPA solution and neutralized with aqueous ammonia to re-separate the crystals. The crystals were filtered, washed with water, dried and recrystallized from a mixture of benzene and n-hexane. Colorless crystals of 2,2-bis[4-(3-aminophenoxy)phenyl]propane thus obtained were 69.2 grams (75% yield) and had a melting point of 106-108°C and their analysis was as follows.
Purity: 99.5% (based on high speed liquid chromatography)

| Elemental analysis (C₂₇H₂₆N₂O₂) | | | |
|---|---|---|---|
| | C | H | N |
| Calculated (%) | 79.02 | 6.34 | 6.83 |
| Found (%) | 79.21 | 6.40 | 6.71 |

MS: 470 (M⁺), 455 (M-CH₃)⁺
IR (KBr.cm⁻¹): 3460 and 3370 (amino group)
1220 (ether linkage)

### Example 5

A 300 ml sealed glass vessel was charged with 50 grams (0.11 mol) of crude 2,2-bis[4-(3-nitrophenoxy)phenyl]propane prepared by the same method as in Example 4, 2.5 grams of 5% Pd/C and 150 ml of ethyleneglycol monomethylether. Hydrogen was introduced at 60-65°C with vigorous stirring. The absorption of hydrogen was stopped after 8 hours at the end of any reaction.

The after-treatment and purification of Example 4 were repeated to afford 36.6 grams (79% yield) of 2,2-bis[4-(3-aminophenoxy)phenyl]propane as colorless crystals having a melting point of 106-108°C and a purity of 99.6% based on high speed liquid chromatography.

### Example 6

The reaction procedure of Example 4 was repeated except 4.85 grams (0.015 mol) of tris(3,6-dioxaheptyl)amine was added and reacted at 150°C for 4 hours.

The same after-treatments as in Example 4 were conducted to afford 705.4 grams (99.8% yield) of 2,2-bis[4-(3-nitrophenoxy)phenyl]propane as yellow brown crystals having a purity of 98% based on liquid chromatography.

### Example 7

A 200 ml glass reaction vessel was charged with 20 grams (0.059 mol) of 2,2-bis(4-hydroxyphenyl)-1,1,1,3,3,3-hexafluoropropane, 24 grams (0.14 mol) of m-dinitrobenzene, 19.4 grams of potassium carbonate and 100 ml of N,N'-dimethylformamide. The mixture was reacted for 7 hours at 140-150°C. At the end of the reaction, the resultant mixture was cooled and poured into 1,000 ml of water to separate crude 2,2-bis[4-(3-nitrophenoxy)phenyl]-1,1,1,3,3,3-hexafluoropropane as tarry matter. The tarry matter was dissolved into benzene and washed with water. The benzene layer was dehydrated with magnesium sulfate and subjected to column chromatography to obtain 28.3 grams (83% yield) of pure 2,2-bis[4-(3-nitrophenoxy)phenyl]-1,1,1,3,3,3-hexafluoropropane as a yellow oil.

The IR spectrum of this compound identified the desired product by the absence of hydroxyl absorption and by the presence of ether absorption.
- IR (NaCl, neat, cm⁻¹):: 1530 and 1350 (nitro group),
1240 (ether linkage)
In the next step, a 300 ml glass reaction vessel was charged with 20 grams (0.035 mol) of crude 2,2-bis[4-(3-nitrophenoxy)phenyl]-1,1,1,3,3,3-hexafluoropropane, 2 grams of active carbon, 0.2 gram of ferric chloride hexahydrate and 100 ml of IPA. The mixture was refluxed for 30 minutes with stirring, 7 grams (0.14 mol) of hydrazine hydrate was added dropwise at 60-70°C during 2 hours, and the mixture then refluxed for a further 5 hours with stirring. The resultant reaction mixture was cooled, filtered to remove the catalyst and heated to distill off 60 ml of IPA. To the residue, 80 grams of 17.5% aqueous hydrochloric acid and 10 grams of sodium chloride were added and the mixture cooled to 20-25°C with stirring. The separated crystals were filtered and recrystallized again from 40 ml of IPA and 80 grams of 17.5% aqueous hydrochloric acid. The resulting crystals were dissolved into 50% aqueous IPA solution and neutralized with aqueous ammonia to re-separate the crystals. The crystals were filtered, washed with water, dried and recrystallized from a solvent mixture of benzene and n-hexane. The colorless crystals of 2,2-bis[4-(3-aminophenoxy)phenyl]-1,1,1,3,3,3-hexafluoropropane thus obtained amounted to 13.6 grams (75% yield) and had a melting point of 137-139°C. The analytical results were as follows.
Purity: 99.2% (based on high speed liquid chromatography)

| Elemental analysis (C₂₇H₂₀N₂F₆) | | | | |
|---|---|---|---|---|
| | C | H | N | F |
| Calculated (%) | 62.55 | 3.85 | 5.41 | 22.00 |
| Found (%) | 62.30 | 3.89 | 5.20 | 21.95 |

IR (KBr.cm⁻¹): 3480 and 3380 (amino group),
1240 (ether linkage)

### Example 8

A 500 ml glass reaction vessel was charged with 20 grams (0.093 mol) of 4,4'-dihydroxybenzophenone, 37.5 grams (0.22 mol) of m-dinitrobenzene, 30 grams of potassium carbonate and 350 ml of sulfolane. The mixture was reacted at 160-170°C for 10 hours. At the end of the reaction, the resultant mixture was poured into 2,000 ml of water and stirred for 30 minutes. The separated crystals were filtered, washed with water and dried to obtain 39 grams (92% yield) of 4,4'-bis(3-nitrophenoxy)benzophenone as brown crystals. A portion of the crude crystals was recrystallized from ethyleneglycol monomethylether to obtain the pure compound as light yellow crystals having a melting point of 189-191°C. The analytical results were as follows:

| Elemental analysis (C₂₅H₁₆N₂O₇) | | | |
|---|---|---|---|
| | C | H | N |
| Calculated (%) | 65.09 | 3.51 | 6.14 |
| Found (%) | 66.07 | 3.42 | 6.04 |

- IR (KBr.cm⁻¹):: 1650 (carbonyl group)
1250 and 1345 (nitro group)
1245 (ether linkage)

In the next step, a 300 ml glass reaction vessel was charged with 25 grams (0.06 mol) of crude 4,4'-bis(3-nitrophenoxy)benzophenone, 2.5 grams of active carbon, 0.25 gram of ferric chloride hexahydrate and 130 ml of ethyleneglycol monomethylether.

The mixture was stirred for 30 minutes at 70-80°C and then 12 grams (0.24 mol) of hydrazine hydrate were added dropwise at 70-80°C during 2 hours and stirred for further 6 hours at 70-80°C. The resultant reaction mixture was cooled, filtered to remove the catalyst, and the solvent was distilled off. The residue was dissolved by warming into the mixture of 13 grams of 35% hydrochloric acid and 60 ml of water, and 6 grams of sodium chloride were added, and cooled to separate the hydrochloride of the product. The hydrochloride was filtered, recrystallized again from 10% aqueous sodium chloride solution and dissolved by warming into 90 grams of 50% aqueous IPA solution. Then 1 gram of active carbon was added to the resulting hydrochloride solution which was filtered and neutralized with aqueous ammonia to separate crystals. The crystals were filtered, washed with water, dried to obtain 19.0 grams (80% yield) of 4,4'-bis(3-aminophenoxy)benzophenone as colorless crystals having a melting point of 142-144°C. The analytical results were as follows.
Purity: 99.3% (based on high speed liquid chromatography)

| Elementary analysis (C₂₅H₂₀N₂O₃) | | | |
|---|---|---|---|
| | C | H | N |
| Calculated (%) | 75.76 | 5.05 | 7.07 |
| Found (%) | 75.60 | 5.20 | 6.99 |

MS: 396(M⁺), 212
IR(KBr.cm⁻¹): 3470 and 3370(amino group)
1625 (carbonyl group)
1220 and 1240 (ether linkage)

### Example 9

A reaction vessel was charged with 20 grams (0.08 mol) of 4,4'-dihydroxydiphenylsulfone, 32.3 grams (0.19 mol) of m-dinitrobenzene, 26.1 grams of potassium carbonate and 150 ml of N,N-dimethylformamide. The mixture was reacted for 15 hours at 145-150°C. At the end of the reaction, the resultant mixture was poured into 2,000 ml of water and stirred for 30 minutes. The separated crystals were filtered, washed with water, and dried to obtain 35.4 grams (90% yield) of 4,4'-bis(3-nitrophenoxy)diphenylsulfone as brown crystals. A portion of the crude crystals was recrystallized from benzene to obtain the pure compound as light yellow crystals having a melting point of 148-150°C. The analytical results were as follows.

**Elemental analysis (C₂₄H₁₆N₂O₈S)**

| | C | H | N | S |
|---|---|---|---|---|
| Calculated (%) | 58.54 | 3.25 | 5.7 | 6.5 |
| Found (%) | 58.69 | 3.12 | 5.6 | 6.6 |

- IR (KBr.cm⁻¹):: 1520 and 1355 (nitro group),
1340 and 1150 (sulfonyl group),
1240 (ether linkage)

In the next step, a 300 ml glass reaction vessel was charged with 25 grams (0.055 mol) of crude 4,4'-bis(3-nitrophenoxy)diphenylsulfone, 2.5 grams of active carbon, 0.25 gram of ferric chloride hexahydrate and 130 ml of ethyleneglycol monomethylether, and the mixture refluxed for 30 minutes with stirring. After adding by dropping 11 grams (0.22 mol) of hydrazine hydrate at 70-80°C during 2 hours, the mixture was stirred for a further 6 hours at 70-80°C, followed by cooling and filtering to remove the catalyst. The solvent was distilled off in a vacuum and then 12 grams of 35% hydrochloric acid and 73 ml of water were added to the residue which was dissolved by warming. Hydrochloride was separated by cooling after the addition of 7 grams of sodium chloride. The hydrochloride was filtered and recrystallized from 10% aqueous sodium chloride solution and then dissolved into 100 ml of 50% squeous IPA solution and filtered after adding 1 gram of active carbon. The filtrate was neutralized with aqueous ammonia to separate crystals which were filtered, washed with water and dried. By recrystallizing from ethanol, 18.8 grams (79% yield) of 4,4'-bis(3-aminophenoxy)diphenylsulfone was obtained as colorless crystals having a melting point of 134-136°C. The analytical results were as follows.
Purity: 99.2% (high speed liquid chromatography)

| Elemental analysis (C₂₄H₂₀N₂O₄S) | | | | |
|---|---|---|---|---|
| | C | H | N | S |
| Calculated (%) | 66.67 | 4.63 | 6.48 | 7.41 |
| Found (%) | 66.68 | 4.66 | 6.41 | 7.56 |

IR (KBr.cm⁻¹): 3480 and 3380 (amino group),
1310 and 1150 (sulfonyl group),
1230 (ether linkage)

### Example 10

A reaction vessel was charged with 218 grams (1 mol) of 4,4'-dihydroxydiphenylsulfide, 403 grams (2.4 mols) of m-dinitrobenzene, 331 grams (2.4 mols) of potassium carbonate and 2.5 l of N,N-dimethylformamide. The mixture was reacted at 145-150°C for 20 hours. At the end of the reaction, the resultant mixture was cooled, filtered and distilled in a vacuum to remove the solvents. The residue was cooled to 65°C, charged with 800 ml of methanol and stirred for an hour. The separated crystals were filtered, washed with methanol and dried to obtain 429 grams (92.3% yield) of 4,4'-bis(3-nitrophenoxy)diphenylsulfide. The crude crystals had a purity of 85% based on liquid chromatography, and were recrystallized from ethyleneglycol monomethylether to obtain the pure compound as light yellow crystals having a melting point of 97-99°C (corr.).

| Elemental analysis (C₂₄H₁₆N₂O₆S) | | | | |
|---|---|---|---|---|
| | C | H | N | S |
| Calculated (%) | 62.60 | 3.47 | 6.09 | 6.96 |
| Found (%) | 62.87 | 3.29 | 6.02 | 6.77 |

- MS (FD):: 460(M⁺)
- IR (KBr.cm⁻¹):: 1510 and 1345 (nitro group),
1230 (ether linkage)

In the next step, a reaction vessel was charged with 428 grams (0.93 mol) of the crude product thus obtained, 22.6 grams of active carbon, 0.9 gram of ferric chloride hexahydrate and 1.5 l of ethyleneglycol monomethylether and the mixture stirred under reflux for 30 minutes, followed by the addition by dropping of 155.2 grams (3.1 mols) of hydrazine monohydrate during 2 hours and further stirring for 3.5 hours under reflux. The resultant mixture was cooled, filtered to remove the catalyst and concentrated in a vacuum. The residue had added to it 205 ml of 35% hydrochloric acid, 1,120 ml of water and 480 ml of IPA and was dissolved by warming, after which 20 grams of active carbon were added, and the mixture hot-filtered.

After adding 112 grams of sodium chloride, the filtrate was cooled to separate hydrochloride crystals. The crystals were filtered and neutralized with aqueous ammonia, and 265 grams (66% yield) of 4,4'-bis(3-aminophenoxy)diphenylsulfide was obtained as colorless crystals having a melting point of 112.4-113.4°C (corr.). The analytical results were as follows.
Purity: 99.9% and better

| Elemental analysis (C₂₄H₂₀N₂O₂S) | | | | |
|---|---|---|---|---|
| | C | H | N | S |
| Calculated (%) | 71.97 | 5.03 | 7.00 | 8.01 |
| Found (%) | 71.90 | 4.54 | 6.92 | 7.72 |

MS (FD): 400 (M⁺)
IR (KBr.cm⁻¹): 3390 and 3300 (amino group),
1220 (ether linkage)

### Example 11

The procedure of Example 10 was repeated except 3.23 grams (0.01 mol) of tris(3,6-dioxaheptyl)amine was added and reacted for 5 hours at 145-150°C.

The same after-treatment as in Example 10 was performed and 453 grams (98.5% yield) of 4,4'-bis(3-nitrophenoxy)diphenylsulfide were obtained as yellow brown crystals. The purity was 95% based on liquid chromatography.

### Example 12

A reaction vessel was charged with 21 grams (0.125 mol) of m-dinitrobenzene, 5.5 grams (0.05 mol) of hydroquinone, 13.8 grams (0.1 mol) of anhydrous potassium carbonate and 100 ml of dimethylsulfoxide. The reaction was conducted at 130-135°C for 7 hours with stirring under nitrogen.

At the end of the reaction, the resultant mixture was poured into 300 ml of water to separate brown crystals. The crystals were filtered, dried, dissolved by warming in 100 ml of benzene, and hot-filtered to remove insoluble matter.

The filtrate was concentrated to separate light brown crystals. The crystals were filtered, washed with hot methanol, and dried to obtain 10.3 grams (58.5% yield) of 1,4-bis(3-nitrophenoxy) benzene, which was recrystallized from benzene to obtain the pure product as light brown prisms having a melting point of 158-160°C.

| Elemental analysis (C₁₈H₁₂N₂O₆) | | | |
|---|---|---|---|
| | C | H | N |
| Calculated (%) | 61.37 | 3.43 | 7.95 |
| Found (%) | 61.74 | 3.39 | 8.03 |

In the next step, a reaction vessel was charged with 7 grams (0.02 mol) of the 1,4-bis(3-nitrophenoxy)benzene obtained above, 0.1 gram of 5% Pd/C catalyst and 25 ml of IPA and hydrogen gas was introduced with vigorous stirring. The reaction was carried out at 60-70°C for 4 hours and hydrogen absorption stopped at 2,700 ml. After the end of the reaction, the resultant mixture was immediately hot-filtered at the same temperature and allowed to cool. 1,4-Bis(3-aminophenoxy)benzene separated as white needles which were filtered, washed and dried to obtain 5.35 grams (91.5% yield) of pure product having a melting point of 126-127°C.

| Elemental analysis (C₁₈H₁₆N₂O₂) | | | |
|---|---|---|---|
| | C | H | N |
| Calculated (%) | 73.95 | 5.52 | 9.58 |
| Found (%) | 73.9 | 5.55 | 9.61 |

### Example 13

A reaction vessel with a stirrer, thermometer, and reflux condenser was charged with 67.1 grams (0.25 mol) of 1-(4-hydroxyphenyl)-1,3,3-trimethyl-6-hydroxyindan, 100.9 grams (0.6 mol) of m-dinitrobenzene, 69.1 grams (0.5 mol) of anhydrous potassium carbonate and 650 ml of N,N-dimethylformamide. The reaction was carried out at 150-153°C for 7 hours with stirring under nitrogen.

After the end of the reaction, the resultant mixture was filtered to remove inorganic salt and concentrated in a vacuum using an evaporator. The brown oily residue thus obtained was mixed with 280 ml of ethyleneglycol monomethylether and 20 ml of water, dissolved by warming, and allowed to cool. The separated crystals were filtered, washed and dried to obtain 112.5 grams (88.1% yield) of 1-[4-(3-nitrophenoxy)phenyl]-1,3,3-trimethyl-6-(3-nitrophenoxy)indan. By recrystallizing from ethanol, the pure product was obtained as light yellow needles having a melting point of 90-92°C.

| Elemental analysis (C₃₀H₂₆N₂O₆) | | | |
|---|---|---|---|
| | C | H | N |
| Calculated (%) | 70.58 | 5.13 | 5.49 |
| Found (%) | 70.62 | 5.18 | 5.43 |

In the next step, a sealed reduction vessel with a stirrer and thermometer was charged with 10.21 grams (0.02 mol) of 1-[4-(3-nitrophenoxy)phenyl]-1,3,3-trimethyl-6-(3-nitrophenoxy)indan obtained as above, 0.3 gram of 5% Pd/C catalyst and 30 ml of ethanol, and hydrogen gas was introduced with vigorous stirring. The reaction was carried out at 62-68°C for 4 hours and hydrogen absorption stopped at 2,760 ml. After the end of the reaction, the resulting mixture was filtered to remove the catalyst and concentrated in an evaporator to recover the solvent.

The concentrated residue was mixed with 6.5 grams of concentrated hydrochloric acid and 50 ml of 20% aqueous IPA solution, and dissolved by warming, active carbon was added and the mixture hot-filtered. The filtrate was dropped into dilute aqueous ammonia to separate precipitate which was filtered, washed and dried. 1-[4(3-aminophenoxy)phenyl]-1,3,3-trimethyl-6-(3-aminophenoxy)indan was obtained in an amount of 8.5 grams (94.3% yield) and had a melting point of 70-72°C. The analytical results were as follows.

| Elemental analysis (C₃₀H₃₀N₂O₂) | | | |
|---|---|---|---|
| | C | H | N |
| Calculated (%) | 79.97 | 6.71 | 6.22 |
| Found (%) | 80.12 | 6.76 | 6.20 |

- NMR Sepctrum:: Solvent: Acetone - D₆
Temperature: room temperature
1.05 ppm ( 3H singlet)
1.35 ppm ( 3H singlet)
1.65 ppm ( 3H singlet)
2.3-2.5 ppm ( 2H multiplet)
3.6-4.2 ppm ( 4H singlet)
6.0-7.3 ppm (15H multiplet)
- MS Spectrum: (M/e)
M⁺ 450, 435, 312, 250, 218

### Example 14

An autoclave was charged with 12.8 grams of 1-[4-(3-nitrophenoxy)phenyl]-1,3,3-trimethyl-6-(3-nitrophenoxy)indan as obtained in Example 13, 1 gram of Raney nickel catalyst and 50 ml of diethyleneglycol dimethylether. Reduction was carried out at 80-90°C for an hour under a hydrogen pressure of 30 kg/cm². The after-treatment of Example 13 was repeated to obtain 9.3 grams (82.5% yield) of the desired product having a melting point of 70-72°C.

### Example 15

A reaction vessel with a stirrer, thermometer, and reflux condenser was charged with 77.1 grams (0.25 mol) of 6,6'-dihydroxy-3,3,3',3'-tetramethyl-1,1'-spirobiindan, 100.9 grams (0.6 mol) of m-dinitrobenzene, 69.1 grams (0.5 mol ) of anhydrous potassium carbonate and 650 ml of N,N-dimethylformamide. The reaction was conducted at 150-153°C for 12 hours with stirring under nitrogen. After the end of the reaction, the resultant mixture was poured into 650 ml of water. The separated light brown precipitate was filtered, washed with IPA and dried. 6,6'-Bis(3-nitrophenoxy)-3,3,3',3'-tetramethyl-1,1'-spirobiindan was obtained in an amount of 116.5 grams (84.6% yield): The pure product was prepared by recrystallizing from ethylalcohol as light brown needles having a melting point of 173.5-175°C.

| Elemental analysis (C₃₃H₃₀N₂O₆) | | | |
|---|---|---|---|
| | C | H | N |
| Calculated (%) | 72.0 | 5.49 | 5.09 |
| Found (%) | 71.83 | 5.39 | 5.10 |

In the next step, a sealed reduction vessel with a stirrer and thermometer was charged with 11 grams (0.02 mol) of 6,6'-bis(3-nitrophenoxy)-3,3,3',3'-tetramethyl-1,1'-spirobiindan, 0.3 g of 5% Pd/C catalyst and 40 ml of ethyleneglycol monomethylether, and hydrogen gas was introduced with vigorous stirring.

The mixture was reacted at 70-80°C for 8 hours and hydrogen absorption stopped at 2,650 ml. At the end of the reaction, the resultant mixture was hot-filtered to remove the catalyst and diluted with 50 ml IPA. The separated crystals were filtered, washed with IPA and dried.

6,6'-Bis(3-aminophenoxy)-3,3,3',3'-tetramethyl-1,1'-spirobiindan was obtained as white needles in an amount of 7.5 grams (76.4% yield) and had a melting point of 205-206°C. The analytical results were as follows.

| Elemental analysis (C₃₃H₃₄N₂O₂) | | | |
|---|---|---|---|
| | C | H | N |
| Calculated (%) | 80.77 | 6.98 | 5.71 |
| Found (%) | 80.62 | 7.05 | 5.70 |

- MMR Spectrum:: Solvent: Acetone-D₆
Temperature: room temperature
1.4 ppm (12H doublet)
2.2-2.5 ppm ( 4H multiplet)
6.0-6.5 ppm ( 8H multiplet)
6.5-7.3 ppm ( 6H multiplet)
- MS Sepctrum: (M/e)
M⁺ 490, 475, 238, 65

### Example 16

The procedure of Example 15 was repeated using 80 grams (0.8 mol) of anhydrous potassium hydrogen carbonate in place of anhydrous potassium carbonate and 800 ml of 1,3-dimethyl-2-imidazolidinon in place of N, N-dimethylformamide. The reaction was conducted at 170-175°C for 10 hours. The intermediate thus obtained, 6,6'-bis(3-nitrophenoxy)-3,3,3',3'-tetramethyl-1,1'-spirobiindan, was 112 grams in amount (81.4% yield).

A reduction vessel was charged with 11 grams of the intermediate compound thus obtained, 0.2 gram of 5% Pt/C catalyst and 100 ml of ethanol, and reduction was conducted by introducing hydrogen under vigorous stirring.

After the end of the reaction, the resultant mixture was filtered to remove the catalyst, and concentrated, followed by addition by dropping of 75 grams of 15% aqueous hydrochloric acid. The separated hydrochloride of the desired product was filtered, washed with water and neutralized with stirring in dilute aqueous ammonia. The separated crystals were filtered, washed with water and dried to obtain 8.75 grams (89.2% yield) of the desired product having a melting point of 205-206°C.

### Example 17

A 1 l glass reaction vessel was charged with 71 grams (0.25 mol) of 2,2-bis (4-hydroxy-3,5-dimethylphenyl)propane, 100.9 grams (0.6mol) of m-dinitrobenzene, 82.8 grms of potassium carbonate and 660 ml of N,N-dimethylformamide. The mixture was reacted at 140-150°C for 15 hours. After the end of the reaction, the resultant mixture was cooled, and filtered to remove potassim nitrite and the solvent was distilled from the filtrate. The residue was cooled to 90°C, 700 ml of water was added and the mixture stirred for an hour. The separated crystals were filtered, and washed with water to obtain crude 2,2'-bis[3,5-dimethyle-4-(3-nitrophenoxy)phenyl]propane as brown crystals.

In the next step, a 1 l glass reaction vessel was charged with the brown crystals above obtained, 13 grams of active carbon, 1.3 grams of ferric chloride hexahydrate and 550 ml of ethyleneglycol monomethylether, and the mixture refluxed for 30 minutes with stirring. Then 50 grams (1.0 mol) of hydrazine hydrate was added by dropping at 70-80°C during 2 hours and stirring was continued for a further 7 hours at 70-80°C. The reaction mixture was cooled, filtered to remove the catalyst and ethyleneglycol monomethylether was distilled off. The residue had added to it successively 250 grams of 30% aqueous hydrochloric acid, 120 ml of methanol and 30 grams of sodium chloride and the mixture was cooled to 20 - 25°C with stirring. The separated cystals were filtered, and dissolved into 560 ml of 50% aqueous methanol solution, 50 grams of sodium chloride were added and the mixture was cooled with stirring to 20-25°C. The separated crystals were filtered, neutralized in water with ammonia, and filtered, washed with water and dried. By recrystallizing from a solvent mixture of toluene and n-heptane, 88.5 grams (76% yield) of 2,2-bis[3,5-dimethyl-4-(3-aminophenoxy)phenyl]propane were obtained as pale brown crystals having a melting point of 112-114°C. The analytical results were as follows.
Purity: 99.5% (based on high speed liquid chromatography)

| Elemental analysis (C₃₁H₃₄N₂O₂) | | | |
|---|---|---|---|
| | C | H | N |
| Calculated (%) | 79.83 | 7.30 | 6.01 |
| Found (%) | 80.15 | 6.90 | 5.85 |

IR(KBr.cm⁻¹): 1220 (ether linkage)
3440 and 3350 (ammonium group)

### Example 18

A 1 l glass reaction vessel was charged with 64 grams (0.25 mol) of 2,2-bis (4-hydroxy-3-methylphenyl)propane, 100.9 grams (0.6 mol)of m-dinitrobenzene, 82.8 grams of potassium carbonate and 650 ml of N,N-dimethylformamide. The mixture was reacted at 145-150°C for 8 hours, cooled at the end of the reaction and poured into 3 l of water. 2,2-Bis[3-methyl-4-(3-nitrophenoxy)phenyl]propane separated as tarry matter. The tarry matter was washed with water.

In the next step, a 1 l glass reaction vessel was charged with the above obtained tarry matter, 12 grams of active carbon, 1.2 grams of ferric chloride hexahydrate and 630 ml of ethyleneglycol monomethylether and the mixture was refluxed for 30 minutes with stirring. The mixture had added to it by dropping 50 grams (1.0 mol) of hydrazine hydrate at 70-80°C during 2 hours and was then stirred for 6 hours under reflux. After the end of the reaction, the resulting mixture was cooled, filtered to remove the catalyst and ethyleneglycol monomethylether was distilled off. The residue was mixed with 500 ml of 20% aqueous methanol solution and 62.5 grams of concentrated hydrochloric acid, and dissolved by warming, after which 60 grams of sodium chloride was added and the mixture cooled to 20-25°C. The separated crystals were filtered and dissolved again by warming in 450 ml of 20% aqueous methanol solution, 45 grams of sodium chloride were added and the mixture cooled with stirring to 20-25°C. The separated crystals were filtered, neutralized in water with aqueous ammonia, filtered again and washed with water. The crystals thus obtained were dissolved by warming in toluene and the separated water layer was removed. Crystals were separated by adding n-heptane to the solution, and were filtered and dried to obtain 79.5 grams (72.6% yield) of 2.2-bis[3-methyl-4-(3-aminophenoxy)phenyl]propane as pale brown crystals having a melting point of 146-148°C. The analytical results were as follows.
Purity : 99.6% (based on high speed liquid chromatagraphy)

| Elemental analysis (C₂₉H₃₀N₂O₂) | | | |
|---|---|---|---|
| | C | H | N |
| Calculated (%) | 79.45 | 6.85 | 6.39 |
| Found (%) | 79.67 | 6.84 | 6.19 |

IR(KBr.cm⁻¹): 1225 (ether linkage)
3470 and 3390 (ammonium group)

### Example 19

A 1 l glass reaction vessel was charged with 60.5 grams (0.25 mol) of 2-(4-hydroxy-3-methylphenyl)-2-(4-hydroxyphenyl)propane, 100.9 grams (0.6 mol) of m-dinitrobenzene, 82.8 grams of potassium carbonate and 650 ml of N,N-dimethylformamide. The mixture was reacted at 140-150°C for 12 hours, cooled after the end of the reaction, and poured into 3 l of water. Pale brown crystals were obtained in an amount of 109 grams (90.0% yield) of crude 2-[3-methyl-4-(3-nitrophenoxy)phenyl]-2-[4-(3-nitrophenoxy)phenyl]propane having a melting point of 114-116°C.

In the second step, a 1 l glass reaction vessel was charged with the above obtained pale brown crystals, 12 grams of active carbon, 1.2 grams of ferric chloride hexahydrate and 600 ml of IPA and the mixture stirred for 30 minutes under reflux. Then 50 grams (1.0 mol) of hydrazine hydrate was dropped into the mixture at 70-80°C during 2 hours, and the mixture refluxed for a further 6 hours with stirring. The resultant mixture was cooled, filtered to remove the catalyst, and ethyleneglycol monomethylether was distilled off. The residue was mixed with 500 ml of 10% aqueous methanol solution and 62.6 grams of concentrated hydrochloric acid, and dissolved by warming, after which 50 grams of sodium chloride was added and the mixture cooled to 20-25°C with stirring. The separated crystals were filtered, mixed with 450 ml of 10% aqueous methanol solution and dissolved by warming, 45 grams of sodium chloride were added and the mixture cooled to 20-25°C with stirring.

The separated crystals were filtered, neutralized with aqueous ammonia in toluene with stirring, and dissolved by warming, with removal of the separated water layer.

n-Heptane was added to the solution. The separated crystals were filtered and dried to obtain 74.8 grams (70.5% yield) of 2-[3-methyl-4-(3-aminophenoxy)phenyl]-2-[4-(3-aminophenoxy)phenyl]propane as pale brown crystals having a melting point of 114-116°C. The analytical results were as follows.
Purity: 99.3% (based on high speed liquid chromatography)

| Elemental analysis (C₂₈H₂₈N₂O₂) | | | |
|---|---|---|---|
| | C | H | N |
| Calculated (%) | 79.25 | 6.60 | 6.60 |
| Found (%) | 79.65 | 6.61 | 6.14 |

IR(KBr.cm⁻¹): 1225 (ether linkage)
3440 and 3360 (ammonium group)

### Example 20

A 1 l glass reaction vessel was charged with 64 grams (0.25 mol) of 2-(4-hydroxy-3,5-dimethylphenyl)-2-(4-hydroxyphenyl)propane, 100.9 grams (0.6 mol) of m-dinitrobenzene, 82.8 grams of potassium carbonate and 630 ml of N,N-dimethylformamide. The mixture was reacted at 140-150°C for 14 hours, cooled after the end of the reaction, and poured into 3,000 ml of water. The pale brown crystals obtained were 106 grams (85.0% yield) of crude 2-[3,5-dimethyle-4-(3-nitrophenoxy)phenyl]-2-[4-(3-nitrophenoxy)phenyl]propane having a melting point of 137-139°C.

In the next step, a 1 l glass vessel was charged with the above obtained pale brown crystals, 12 grams of active carbon, 1.2 grams of ferric chloride hexahydrate and 630 ml of ethyleneglycol monomethylether, and the mixture refluxed for 30 minutes with stirring. Then 50 grams (1.0 mol) of hydrazine hydrate was added by dropping during 2 hours, and the mixture stirred for a further 8 hours under reflux, cooled, and filtered to remove the catalyst, and ethyleneglycol monomethylether was distilled off. The residue was mixed with 500 ml of 25% aqueous methanol solution and 62.5 grams of concentrated hydrochloric acid, and dissolved by warming, and 50 grams of sodium chloride were added and the mixture cooled to 20-25°C with stirring. The separated crystals, were filtered, mixed with 400 ml of 25% aqueous methanol solution, and dissolved by warming, and 40 grams of sodium chloride were added and the mixture cooled to 20-25°C with stirring. The separated crystals were filtered, neutralized in water with aqueous ammonia, filtered again, and washed with water. The crystals thus obtained were dissolved in toluene by warming and the separated water layer was removed. n-Heptane was added to the solution. The separated crystals were filtered and dried. The pale brown crystals obtained were 82.1 grams (75% yield) of 2-[3,5-dimethyl-4-(3-aminophenoxy)phenyl]-2-[4-(3-aminophenoxy)phenyl]propane having a melting point of 137-139°C. Results of analysis were as follows.
Purity: 99.2% (based on high speed liquid chromatography)

| Elemental analysis (C₂₉H₃₀N₂O₂) | | | |
|---|---|---|---|
| | C | H | N |
| Calculated (%) | 79.45 | 6.85 | 6.39 |
| Found (%) | 79.65 | 6.62 | 5.98 |

IR(KBr.cm⁻¹): 1230 (ether linkage)
3450 and 3370 (ammonium group)

## Claims

1. A method for preparing a bis(3-aminophenoxy) derivative of an aromatic or bridged aromatic hydrocarbon represented by the following general formula: 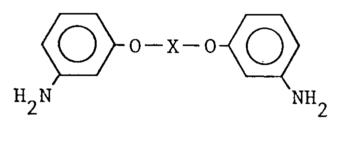 where X is 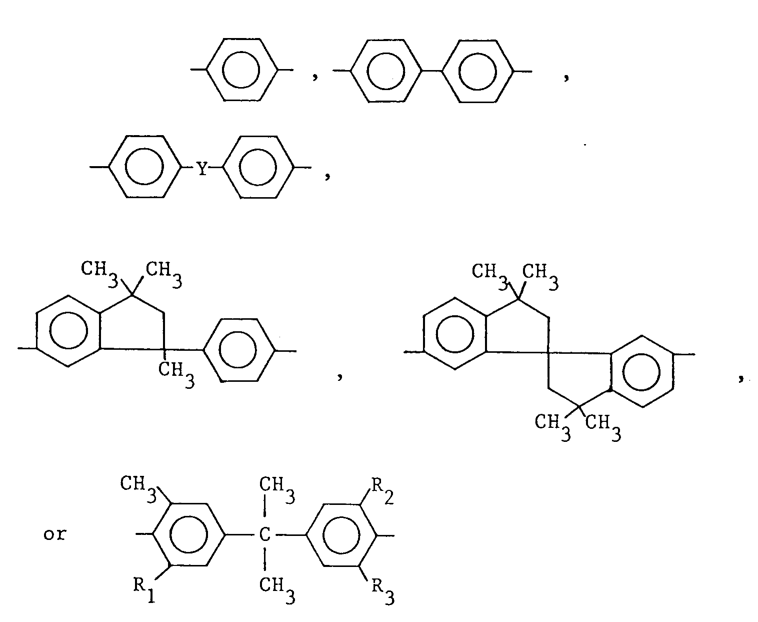 where Y is C₁-C₁₀ hydrocarbon, -C(CF₃)₂-, -CO-, -S-, -SO-, -SO₂-, or -O-,
and R₁, R₂, R₃ are H, or CH₃,
which comprises conducting a condensation reaction of m-dinitrobenzene in a dipolar aprotic solvent in the presence of a base with a dihydroxy derivative of an aromatic or bridged aromatic hydrocarbon represented by the following general formula:
HO-X-OH
where X is the same as above,
and reducing the resultant bis(3-nitrophenoxy) derivative of the aromatic or bridged aromatic hydrocarbon represented by the following general formula (II): 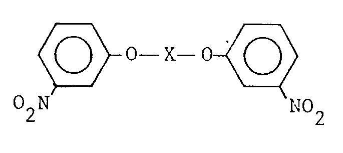 where X is the same as above.

2. The method of claim 1, wherein said condensation reaction is conducted in the presence of a nitrogen-containing aliphatic polyether reaction accelerator represented by the following general formula:
N[-CH₂-CH₂-O(-CH₂-CH₂-O)ₙ-R]₃
where R is C₁-C₄ alkyl and n is the integer 1 or 2.

3. The method of claim 1 or claim 2, wherein said base is a carbonate or hydrogen carbonate of an alkali metal.

4. The method of any preceding claim, wherein said reducing reaction is conducted with hydrazine.

5. The method of any of claims 1 to 3, wherein said reducing reaction is conducted with hydrogen in the presence of a catalyst.

6. The method of any preceding claim, wherein said dihydroxy derivative is represented by the following general formula: 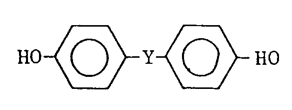 where Y is a C₁-C₁₀ hydrocarbon, -C(CF₃)₂-, -CO-, -S-, -SO-, -SO₂-, or -O-.

7. The method of any of claims 1 to 5, wherein said dihydroxy derivative is hydroquinone.

8. The method of any of claims 1 to 5, wherein said dihydroxy derivative is 4,4'-dihydroxybiphenyl.

9. The method of any of claims 1 to 5, wherein said dihydroxy derivative is 1-(4-hydroxyphenyl)-1,3,3-trimethyl-6-hydroxyindan.

10. The method any of claims 1 to 5, wherein said dihydroxy derivative is 6,6'-dihydroxy-3,3,3',3'-tetramethyl-1,1'-spirobiindan.

11. The method of any of claims 1 to 5, wherein said dihydroxy derivative is a 2,2-bis(4-hydroxyphenyl)propane derivative represented by the following general formula: 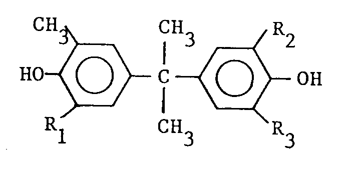 where R₁_{,} R₂ and R₃ are H, or Ch₃.

12. 4,4'-Bis(3-aminophenoxy)biphenyl.

13. 1-[4-(3-Aminophenoxy)phenyl]-1,3,3-trimethyl-6-(3-aminophenoxy)indan.

14. 6,6'-Bis(3-aminophenoxy)-3,3,3',3'-tetramethyl-1,1'-spirobiindan.

15. A methyl substituted 2,2-bis[4-(3-aminophenoxy)phenyl]propane represented by the following general formula: 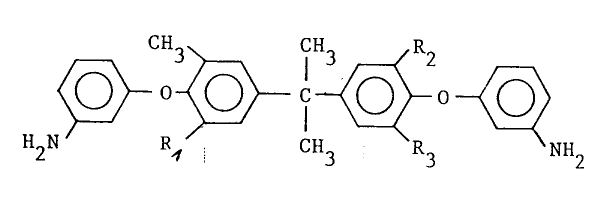 where R₁, R₂ and R₃ are H, or CH₃.

## Patentansprüche

1. Ein Verfahren zur Herstellung eines Bis(3-Aminophenoxy) Derivates eines aromatischen oder eines aromatischen Brücken-Kohlenwasserstoffs, welcher durch die folgende Formel beschrieben wird: 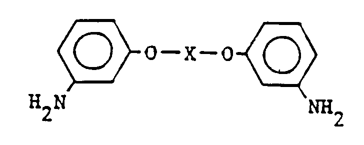 Wobei X ist 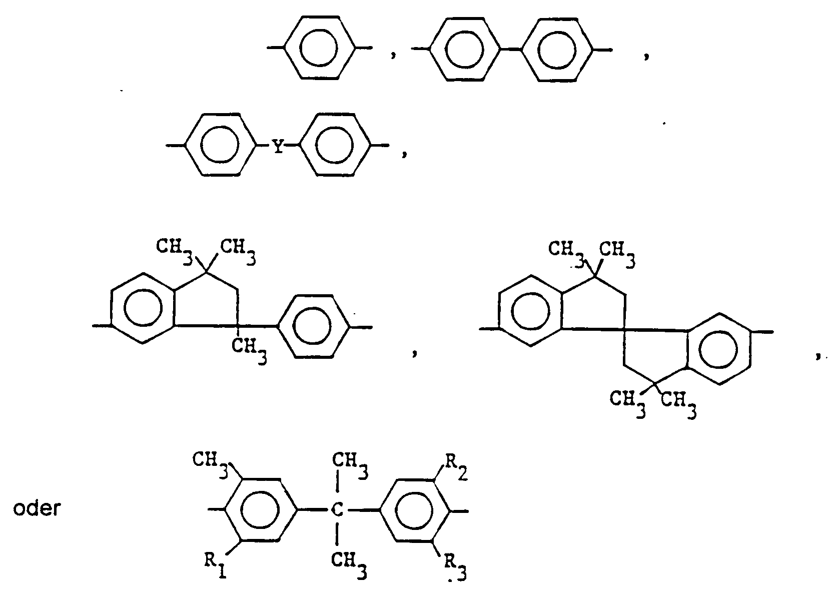 wobei Y ein C₁-C₁₀ Kohlenwasserstoff, -C(CF₃)₂-, -CO-, -S-,-SO-, -SO₂- oder -O- ist und wobei R₁, R₂ und R₃ H oder CH₃ sind,
das überführend eine Kondensationreaktion eines m-Dinitro-Benzens in einer dipolaren aprotischen Lösung im Beisein einer Base mit einem Dihydroxy Derivat eines aromatischen oder aromatischen Brücken-Kohlenwasserstoffes umfaßt, beschrieben durch die folgende Formel:
HO-X-OH
wobei X das gleiche wie oben ist,
und die Reduzierung des resultierenden Bis(3-Nitrophenoxy) Derivats von dem aromatischen oder aromatischen Brücken-Kohlenwasserstoffs umfaßt, beschrieben durch die folgende allgemeine Formel (II): 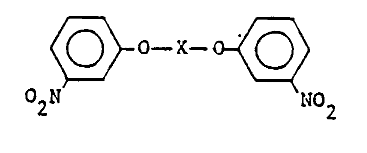 wobei X das gleiche wie oben ist.

2. Das Verfahren nach Anspruch 1, wobei diese Kondensationsreaktion im Beisein eines Stickstoffs überführt wird, einen aliphatischen Polyether-Reaktionsbeschleuniger enthaltend; beschrieben durch die folgende allgemeine Formel:
N[-CH₂-CH₂-O(-CH₂-CH₂-O)ₙ-R]₃
wobei R ein C₁-C₄ Alkyl ist und n die ganze Zahl 1 oder 2 ist.

3. Das Verfahren nach Anspruch 1 oder 2, wobei die Base ein Karbonat oder ein Hydrogenkarbonat eines Alkali-Metalls ist.

4. Das Verfahren nach einem der folgenden Ansprüche, wobei die Reduktionsreaktion mit Hydrazin geleitet wird.

5. Ein Verfahren nach einem der Ansprüche 1 bis 3, wobei die Reduktionsreaktion durch Hydrogen geleitet wird unter Anwesenheit eines Katalysatoren.

6. Ein Verfahren nach einem der folgenden Ansprüche, wobei das Dihydroxy Derivat durch die folgende allgemeine Formel beschrieben wird: 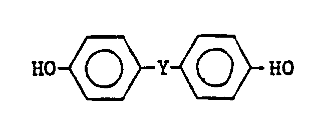 wobei Y ein C₁-C₁₀ Kohlenwasserstoff, -C(CF₃)₂-, -CO-, -S-,-SO-, -SO₂- oder -O- ist.

7. Das Verfahren nach einem der Ansprüche 1 bis 5, wobei das Dihydroxy Derivat Hydroquinon ist.

8. Das Verfahren nach einem der Ansprüche 1 bis 5, wobei das Dihydroxy Derivat 4,4'-Dihydroxybiphenyl ist.

9. Das Verfahren nach einem der Ansprüche 1 bis 5, wobei das Dihydroxy Derivat 1-(4-hydroxyphenyl)-1,3,3-trimethyl-6-hydroxyindan ist.

10. Das Verfahren nach einem der Ansprüche 1 bis 5, wobei das Dihydroxy Derivat 6,6'-dihydroxy-3,3,3',3'-tetramethyl-1,1'-spirobiindan ist.

11. Das Verfahren nach einem der Ansprüche 1 bis 5, wobei das Dihydroxy Derivat ein 2,2-bis(4-hydroxyphenyl)propan Derivat ist, beschrieben durch die folgende allgemeine Formel: 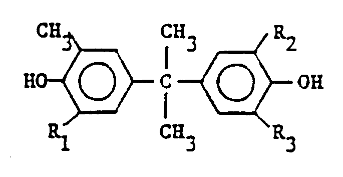 wobei R₁,R₂ und R₃ H oder CH₃ sind.

12. 4,4'-Bis(3-aminophenoxy)biphenyl.

13. 1-[4-(3-Aminophenoxy)phenyl]-1,3,3-trimethyl-6-(3-aminophenoxy)-indan.

14. 6,6'-Bis(3-aminophenoxy)-3,3,3',3'-tetramethyl-1,1'-spirobiindan.

15. Ein Methyl substituiertes 2,2-bis[4-(3-aminophenoxy)phenyl]propan, beschreiben durch die folgende allgemeine Formel: 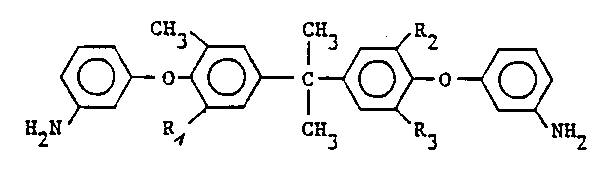 wobei R₁,R₂ und R₃ H oder CH₃ sind.

## Revendications

1. Procédé pour la préparation d'un dérivé bis(3-aminophénoxy) d'un hydrocarbure aromatique ou d'un hydrocarbure aromatique ponté représenté par la formulation générale suivante : 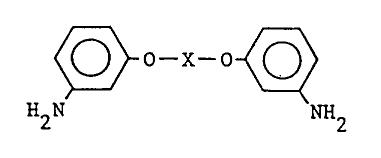 où X est 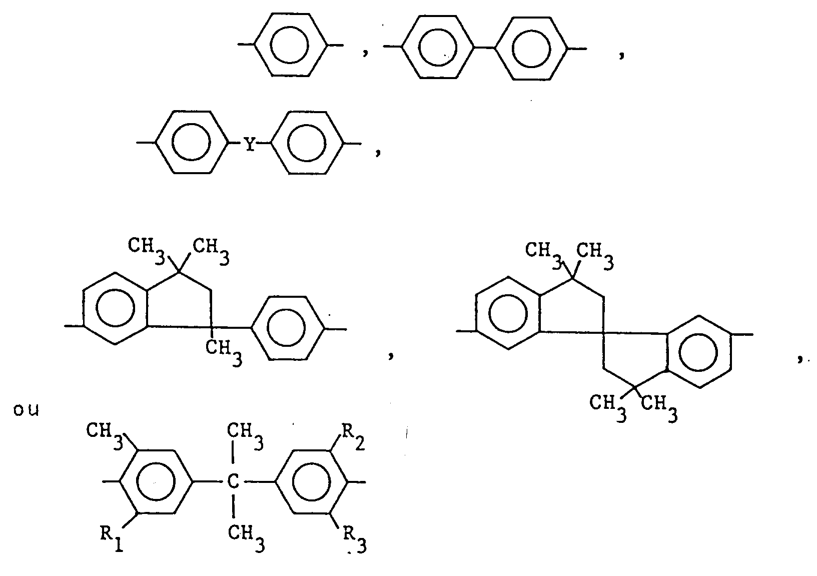 où Y est un hydrocarbure en C₁-C₁₀, -C(CF₃)₂-, -CO-, -S-, -SO-, -SO₂-, ou -O-,
et R₁, R₂, R₃ sont H ou CH₃,
consistant à effectuer une réaction de condensation de m-dinitro-benzène dans un solvant dipolaire aprotique en présence d'une base contenant un dérivé dihydroxy d'un hydrocarbure aromatique ou d'un hydrocarbure aromatique ponté représenté par la formulation générale suivante :
HO-X-OH
où X est le même que ci-dessus,
et à réduire le dérivé bis(3-nitrophénoxy) résultant de l'hydrocarbure aromatique ou aromatique ponté représenté par la formulation générale suivante (II) : 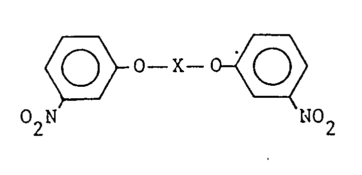 où X est le même que ci-dessus.

2. Procédé selon la revendication 1, caractérisé en ce ladite réaction de condensation est effectuée en présence d'un accélérateur de réaction de polyéther aliphatique contenant de l'azote et représenté par la formulation générale suivante :
N[-CH₂-CH₂-O(-CH₂-CH₂-O)ₙ-R]₃
où R est un alkyle en C₁-C₄ et n est un nombre entier, 1 ou 2.

3. Procédé selon la revendication 1 ou la revendication 2, caractérisé en ce que ladite base est un carbonate ou un carbonate d'hydrogène d'un métal alcalin.

4. Procédé selon l'une quelconque des revendications précédentes, caractérisé en ce que la réaction de réduction est effectuée avec de l'hydrazine.

5. Procédé selon l'une quelconque des revendications 1 à 3, caractérisé en ce que ladite réaction de réduction est effectuée avec de l'hydrogène en présence d'un catalyseur.

6. Procédé selon l'une quelconque des revendications précédentes, caractérisé en ce que ledit dérivé dihydroxy est représenté par la formulation générale suivante : 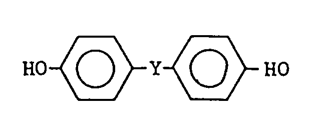 où Y est un hydrocarbure en C₁-C₁₀, -C(CF₃)₂-, -CO-, -S-, -SO-, -SO₂-, ou -O-.

7. Procédé selon l'une quelconque des revendications 1 à 5, caractérisé en ce que ledit dérivé dihydroxy est l'hydroquinone.

8. Procédé selon l'une quelconque des revendications 1 à 5, caractérisé en ce que ledit dérivé dihydroxy est le 4,4'-dihydroxybiphényle.

9. Procédé selon l'une quelconque des revendications 1 à 5, caractérisé en ce que ledit dérivé dihydroxy est le 1-(4-hydroxyphényl)-1,3,3-triméthyl-6-hydroxyindane.

10. Procédé selon l'une quelconque des revendications 1 à 5, caractérisé en ce que ledit dérivé dihydroxy est le 6,6'-dihydroxy-3,3,3',3'-tétraméthyl-1,1'-spirobiindane.

11. Procédé selon l'une quelconque des revendications 1 à 5, caractérisé en ce que ledit dérivé dihydroxy est un dérivé de 2,2-bis(4-hydroxyphényl)propane représenté par la formulation générale suivante : 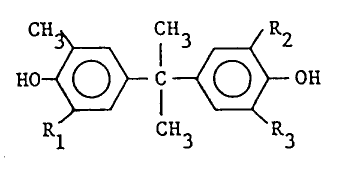 où R₁, R₂ et R₃ sont H ou CH₃.

12. 4,4'-bis(3-aminophénoxy)biphényle.

13. 1-[4-(3-aminophénoxy)phényle]-1,3,3-triméthyl-6-(3-aminophénoxy)-indane.

14. 6,6'-bis(3-aminophénoxy)-3,3,3',3'-tétraméthyl-1,1'-spirobiindane.

15. 2,2'-bis[4-(3-aminophénoxy)phényl]propane méthyle-substitué représenté par la formulation générale suivante : 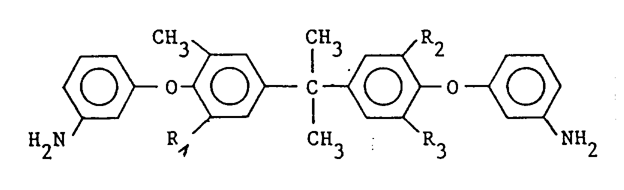 où R₁, R₂ et R₃ sont H ou CH₃.
